# EUROPÄISCHE PATENTSCHRIFT

(11) **EP 1 551 829 B1**
(45) Veröffentlichungstag und Bekanntmachung des Hinweises auf die Patenterteilung: **07.04.2010**
(21) Anmeldenummer: 03808720.1
(22) Anmeldetag: 09.10.2003
(51) Int. Cl.: C07D 407/14, C07D 401/06, C07D 413/14, C07D 417/14, C07D 407/12, C07D 417/12, C07D 239/72, C07D 471/04, C07D 401/12, C07D 413/12, C07D 491/04, C07D 215/20, C07D 239/88, C07D 451/04

(54) **NEUE VERBINDUNGEN MIT ANTIBAKTERIELLER AKTIVITÄT**
NOVEL COMPOUNDS WITH ANTIBACTERIAL ACTIVITY
NOUVEAUX COMPOSES ANTIBACTERIENS

(30) Priorität: 10.10.2002 DE 10247233; 02.12.2002 DE 10256405
(43) Veröffentlichungstag der Anmeldung: 13.07.2005
(73) Patentinhaber: Morphochem Aktiengesellschaft Für Kombinatorische Chemie, 81379 München (DE)
(72) Erfinder: SURIVET, Jean-Philippe, F-68300 Saint-Louis (FR); ZUMBRUNN, Cornelia, CH-4058 Basel (CH); HUBSCHWERLEN, Christian, F-68480 Durmenach (FR); PEREZ FRUTOS HÖNER, Annabelle, 4056 Basel (CH)
(74) Vertreter: Forstmeyer, Dietmar
(86) Internationale Anmeldenummer: PCT/EP2003/011203
(87) Internationale Veröffentlichungsnummer: WO 2004/035569

(56) Entgegenhaltungen:
- WO-A-00/78748
- WO-A-01/07432
- WO-A-01/46150
- WO-A-02/08224
- WO-A-96/39145
- WO-A-99/37635
- US-A- 3 184 462

## Beschreibung

In vielen Ländern der Welt hat die Resistenz gegenüber den derzeit gebräuchlichen Antibiotika in den letzten Jahren beträchtlich zugenommen und zum Teil bedrohliche Ausmasse angenommen. Das Hauptproblem dabei ist, dass diese Erreger nicht nur eine, sondern in der Regel mehrfache Resistenzen tragen. Dies gilt insbesondere für einige Gram-positive Erregergruppen, wie Staphylokokken, Pneumokokken und Enterokokken (S. Ewig et al.; Antibiotika-Resistenz bei Erregern ambulant erworbener Atemwegsinfektionen; Chemother. J. 2002, 11, 12-26; F. Tenover; Development and spread of bacterial resistance to antimicrobial agents: an overview; Clin. Infect. Dis. 2001 Sep 15, 33 Suppl. 3, 108-115)

Eine lange befürchtete Entwicklung ist kürzlich eingetreten: In den USA wurde der erste Stamm von *Staphylococcus aureus* beschrieben, welcher nicht nur Methicillin-resistent, sondern auch gegen Vancomycin hochresistent ist (Centers for Disease Control and Prevention; Staphylococcus aureus resistant to vancomycin - United States, 2002; MMWR 2002, 51, 565-567).

Neben hygienischen Massnahmen in Krankenhäusern sind daher auch verstärkt Anstrengungen erforderlich, neue Antibiotika zu finden, die möglichst eine neue Struktur und einen neuen Wirkungsmechanismus besitzen, um gegen diese Problemkeime wirksam zu sein.

Chinolinderivate mit antibakterieller Wirksamkeit sind in der Publikation WO 02/08224 beschrieben.

Die vorliegende Anmeldung beschreibt eine neuartige Verbindung mit antibakterieller Aktivität.

Die vorliegende Erfindung betrifft eine Verbindung der Formel (I):

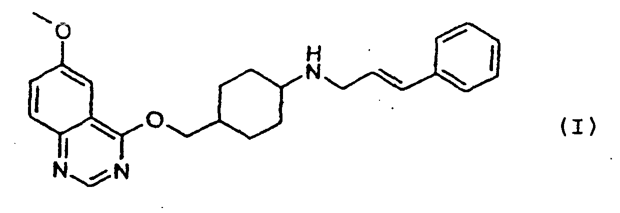

Die Verbindung der Formel (I) enthält aufgrund ihrer Substitution mehrere Chiralitätszentren. Die vorliegende Erfindung umfasst daher sowohl alle reinen Enantiomere und alle reinen Diastereomere, als auch deren Gemische in jedem Mischungsverhältnis. Des weiteren sind von der vorliegenden Erfindung auch alle *cis*/*trans*-Isomeren der Verbindung der allgemeinen Formel (I) sowie Gemische davon umfasst. Des weiteren sind von der vorliegenden Erfindung alle tautomeren Formen der Verbindung der Formel (I) umfasst.

Die therapeutische Verwendung der Verbindung der Formel (I), ihrer pharmakologisch akzeptablen Salze bzw. Solvate und Hydrate sowie Formulierungen und pharmazeutischen Zusammensetzungen liegt ebenfalls im Rahmen der vorliegenden Erfindung.

Die pharmazeutischen Zusammensetzungen gemäß der vorliegenden Erfindung enthalten mindestens eine Verbindung der Formel (I) als Wirkstoff und fakultativ Trägerstoffe und/oder Adjuvantien.

Beispiele für pharmakologisch akzeptable Salze der Verbindung der Formel (I) sind Salze von physiologisch akzeptablen Mineralsäuren wie Salzsäure, Schwefelsäure und Phosphorsäure oder Salze von organischen Säuren wie Methansulfonsäure, p-Toluolsulfonsäure, Milchsäure, Essigsäure, Trifluoressigsäure, Zitronensäure, Bernsteinsäure, Fumarsäure, Oxalsäure, Maleinsäure und Salicylsäure. Weitere Beispiele für pharmakologisch akzeptable Salze der Verbindung der Formel (I) sind Alkali- oder Erdalkalisalze wie z. B. Natrium, Kalium, Lithium, Calcium oder Magnesium Salze, Ammoniumsalze oder Salze von organischen Basen wie z. B. Methylamin, Dimethylamin, Triethylamin, Piperidin, Ethylendiamin, Lysin, Cholinhydroxid, Meglumin, Morpholin oder Arginin Salze. Die Verbindung der Formel (I) kann solvatisiert, insbesondere hydratisiert sein. Die Hydratisierung kann z.B. während des Herstellungsverfahrens oder als Folge der hygroskopischen Natur der anfänglich wasserfreien Verbindung der Formel (I) auftreten.

Auch die Verwendung dieses Wirkstoffs zur Herstellung von Arzneimitteln ist Gegenstand der vorliegenden Erfindung. Im allgemeinen wird die Verbindung der Formel (I) unter Anwendung der bekannten und akzeptablen Modi, entweder einzeln oder in Kombination mit einem beliebigen anderen therapeutischen Mittel verabreicht. Solche therapeutisch nützlichen Mittel können auf einem der folgenden Wege verabreicht werden: oral, z.B. als Dragees, überzogene Tabletten, Pillen, Halbfeststoffe, weiche oder harte Kapseln, Lösungen, Emulsionen oder Suspensionen; parenteral, z.B. als injizierbare Lösung; rektal als Suppositorien; durch Inhalation, z.B. als Pulverformulierung oder Spray, *trans*dermal oder intranasal. Zur Herstellung solcher Tabletten, Pillen, Halbfeststoffe, überzogenen Tabletten, Dragees und harten Gelatinekapseln kann das therapeutisch verwendbare Produkt mit pharmakologisch inerten, anorganischen oder organischen Arzneimittelträgersubstanzen vermischt werden, z.B. mit Lactose, Sucrose, Glucose, Gelatine, Malz, Silicagel, Stärke oder Derivaten derselben, Talkum, Stearinsäure oder ihren Salzen, Trockenmagermilch und dgl. Zur Herstellung von weichen Kapseln kann man Arzneimittelträgerstoffe wie z.B. pflanzliche Öle, Petroleum, tierische oder synthetische Öle, Wachs, Fett, Polyole einsetzen. Zur Herstellung von flüssigen Lösungen und Sirups kann man Arzneimittelträgerstoffe wie z.B. Wasser, Alkohole, wäßrige Salzlösung, wäßrige Dextrose, Polyole, Glycerin, pflanzliche Öle, Petroleum, tierische oder synthetische Öle verwenden. Für Suppositorien kann man Arzneimittelträgerstoffe wie z.B. pflanzliche Öle, Petroleum, tierische oder synthetische Öle, Wachs, Fett und Polyole verwenden. Für Aerosol-Formulierungen kann man komprimierte Gase, die für diesen Zweck geeignet sind, wie z.B. Sauerstoff, Stickstoff und Kohlendioxid einsetzen. Die pharmazeutisch verwendbaren Mittel können auch Zusatzstoffe zur Konservierung, Stabilisierung, Emulgatoren, Süßstoffe, Aromastoffe, Salze zur Veränderung des osmotischen Drucks, Puffer, Umhüllungszusatzstoffe und Antioxidantien enthalten.

Kombinationen mit anderen therapeutischen Mitteln können andere antimikrobielle und antifungale Wirkstoffe beinhalten.

Zur Vorbeugung und/oder Behandlung von Bakterieninfektionen kann die Dosis der erfindungsgemäßen biologisch aktiven Verbindung innerhalb breiter Grenzen variieren und kann auf den individuellen Bedarf eingestellt werden. Im allgemeinen ist eine Dosis von 10 mg bis 4000 mg pro Tag geeignet, wobei eine bevorzugte Dosis 50 bis 3000 mg pro Tag ist. In geeigneten Fällen kann die Dosis auch unter oder über den oben angegebenen Werten liegen. Die tägliche Dosis kann als einfache Gabe oder in mehrfachen Gaben verabreicht werden. Eine typische Einzeldosis beinhaltet etwa 50 mg, 100 mg, 250 mg, 500 mg, 1 g oder 2 g des Wirkstoffs.

### BEISPIELE

### Vergleichsbeispiel: (2,3-Dihydro-benzo[1,4]dioxin-6-ylmethyl)-[4-(6-methoxy-chinazolin-4-yloxymethyl)-cyclohexyl] -amin

### V.a) 4-(6-Methoxy-chinazolin-4-yloxymethyl)-cyclohexanol

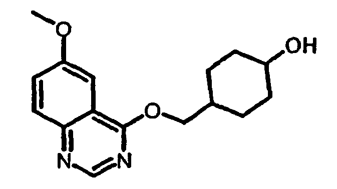

Zu einer Lösung von 4-Hydroxymethyl-cyclohexanol (3g, 12 mmol, synthetisiert analog J.Org.Chem. 1994 59 p. 2748-2761) in DMF (30mL) wurde bei OC Natriumhydrid (79 mg (1.5 eq) gegeben. Nach 20 minuten, wurde eine Lösung von 4-Chloro-6-methoxy-chinazolin (1.94 g, 10mmol)) in DMF (10mL) zugegeben und das Reaktionsgemisch auf 40°C erwärmt. Nach weiteren 2h wurde das Reaktionsgemisch zur Trockne eingeengt. Der Rückstand wurde mit Wasser (100mL) und Essigester (100 mL) verdünnt, die Phasen getrennt und die wässrige Phase zweimal mit je 50mL Essigester extrahiert. Die vereinten organischen Phasen wurden über MgSO₄ getrocknet, filtriert und zur Trockne einrotiert. Der Rückstand wurde mittels Säulenchromatographie an Kieselgel (EtOAc-Hexan 1-2) gereinigt.
Ausbeute: 2.01g, 7 mmol (54%)
MS (EI) m/z 289 [M+H]⁺

### V.b) 4-(6-Methoxy-chinazolin-4-yloxymethyl)-cyclohexanon

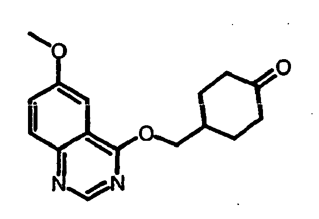

Zu einer Lösung von 4-(6-Methoxy-chinazolin-4-yloxy-methyl)-cyclohexanol (2.88 g, 10mmol) in DMSO (30mL) wurden bei 0C Triethylamin (10mL, 71 mmol) und dann portionenweise Pyr.SO3 (5.8 g, 36mmol) zugegeben. Das Reaktionsgemisch wurde 15 Minuten bei dieser Temperatur gerührt und dann auf Raumtemperatur kommen gelassen. Nach 2h wurde Wasser (300mL) zugegeben. Die wässrige Phase wurde mit Ether (3 x 150mL) extrahiert. Die vereinten organischen Phasen wurden mit ges. Kochsalzlösung gewaschen, über MgSO₄ getrocknet und eingeengt. Das Rohprodukt wurde mittels Chromatographie an Kieselgel (EtOAc-Hex 1-2) gereinigt.
Ausbeute: 2.6g, 9.1 mmol
MS (EI) m/z 287.1 [M+H]⁺

### V.c) Benzyl-[4-(6-methoxy-chinazolin-4-yloxymethyl)-cyclohexyl]-amin

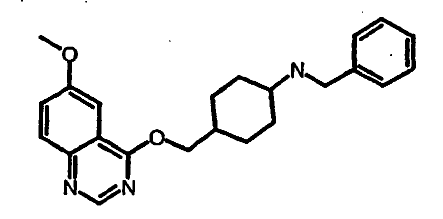

Eine Lösung von 4-(6-Methoxy-chinazolin-4-yloxymethyl)-cyclohexanon (2.87g, 10 mmol) in Methanol (50 ml) wurde mit Benzylamin (1.08mL, 10 mmol) und Natriumcyanoborhydrid (2.2 g, 11 mmol) versetzt. Das Gemisch wurde über Nacht bei Raumtemperatur gerührt. Das Reaktionsgemisch wurde eingeengt und der Rückstand in gesättigter Kaliumcarbonat Lösung und Essigester gelöst. Die wässrige Phase wurde mit Essigester (2x50ml) extrahiert, die vereinten organischen Phasen über Magnesiumsulfat getrocknet und eingeengt. Der Rückstand wurde mittels Säulenchromatographie an Kieselgel (EtOAc-dann EtOAc-MeOH 9/1) gereinigt.
Ausbeute: 2.87g, 10 mmol als *cis*/*trans* Gemisch
MS (EI) m/z 287.1 [M+H]⁺

### V.d) 4-(6-Methoxy-chinazolin-4-yloxymethyl)-cyclohexylamin

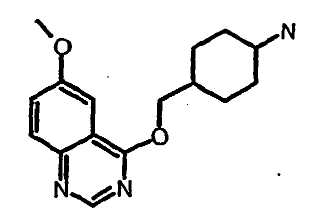

Zu einer Lösung von Benzyl-[4-(6-methoxy-chinazolin-4-yloxymethyl)-cyclohexyl]-amin (2g, 5.3 mmol) in MeOH (30mL) wurde 20% Pd(OH)₂ auf Kohle (1g) zugegeben und unter Wasserstoffatmosphäre (lbar) hydriert bei 65°C. Die Reaktionsmischung wurde abfiltriert und das Filtrat eingeengt.
Ausbeute: 1.44g, 5 mmol als *cis*/*trans* Gemisch
MS (EI) m/z 288 [M+H]⁺

### V.e) Titelverbindung

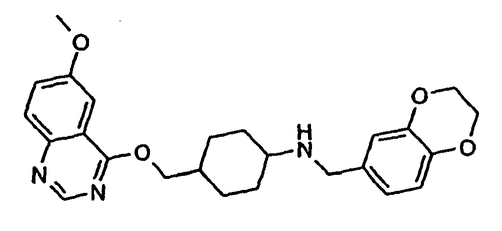

Zu einer Lösung von 4-(6-Methoxy-chinazolin-4-yloxy-methyl)-cyclohexylamin (0.07g, 0.25mmol) in Dichlorethan (1mL) wurde 2,3-Dihydro-benzo[1,4]dioxin-6-carbaldehyd (0.045g, 0.27mmol) und danach Natriumtriacetoxyborhydrid (0.08g, 0.377 mmol) zugegeben. Das Reaktionsgemisch wurde 2 Stunden gerührt dann über Hydromatrix (benetzt mit NaHCO₃ Lösung, 2mL) filtriert und mit Dichlormethan nachgewaschen. Das Filtrat wurde zur Trockne einrotiert. Der Rückstand wurde mittels Säulenchromatographie an Kieselgel (Dichlormethan/MeOH 9/1 dann Dichlormethan/MeOH 6/1) gereinigt.
Ausbeute:0.02g (0.046mmol) als *cis*/*trans*-Gemisch
MS (EI) m/z 436 [M+H]⁺

### Beispiel: [4-(6-Methoxy-chinazolin-4-yloxymethyl)-cyclohexyl]-(3-phenyl-allyl)-amin

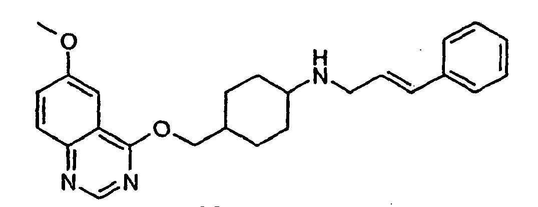

In Analogie zu Beispiel V.e wurde [4-(6-Methoxy-chinazolin-4-yloxymethyl)-cyclohexyl]-(3-phenyl-allyl)-amin in 25% Ausbeute als *cis*/*trans* Gemisch hergestellt (MS (EI) m/z 405 [M+H]⁺).

### Antibakterielle Aktivität:

Die MHK (ug/ml) von Verbindung (I) wurde gegen folgende Bakterienstämme gemessen: S. aureus ATCC 29213, S. aureus 16, E. faecalis ATCC 29212, E. faecium vanA E25-1, H. influenzae 11, E. coli ATCC 25922, M. catarrhalis 117, S. pneumoniae ATCC 49619.

Verbindung (I) hat eine MHK<=0.125 gegen mindestens einen der aufgelisteten Stämme.

## Patentansprüche

1. Verbindung der Formel (I):

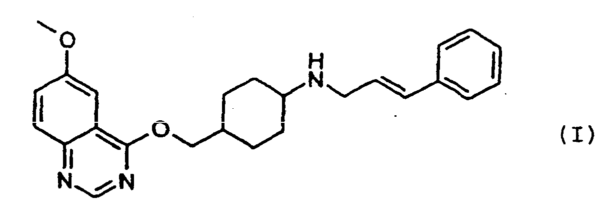

2. Pharmazeutische Zusammensetzungen, die eine Verbindung nach Anspruch 1 und fakultativ Trägerstoffe und/oder Adjuvantien enthalten.

3. Verwendung einer Verbindung oder einer pharmazeutischen Zusammensetzung nach Anspruch 1 oder 2 zur Herstellung eines Medikaments zur Behandlung von Bakterieninfektionen.

## Claims

1. Compound of formula (I):

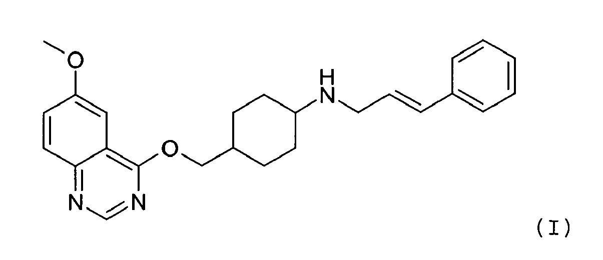

2. Pharmaceutical compositions containing a compound according to claim 1 and optionally carriers and/or adjuvants.

3. Use of a compound or a pharmaceutical composition according to claim 1 or 2 for the manufacture of a medicament for the treatment of bacterial infections.

## Revendications

1. Composé de formule (I):

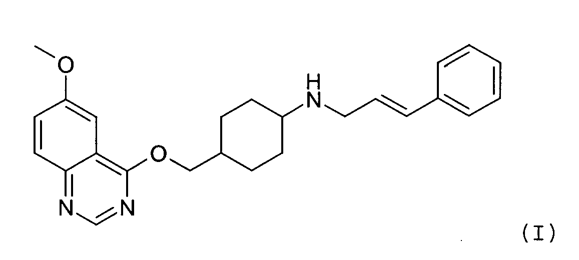

2. Compositions pharmaceutiques contenant un composé selon la revendication 1 et facultativement des supports et/ou des adjuvants.

3. Utilisation d'un composé ou d'une composition pharmaceutique selon les revendications 1 ou 2, pour la fabrication d'un médicament destiné au traitement des infections bactériennes.
